# EUROPEAN PATENT APPLICATION

(11) **EP 3 815 721 A1**
(43) Date of publication of application: **05.05.2021**
(21) Application number: 20163774.1
(22) Date of filing: 17.03.2020
(51) Int. Cl.: A61L 29/04, A61L 29/16, A61K 38/16, A01N 63/00, A01N 63/20

(54) **USE OF A TAIL FIBER PROTEIN FOR PREVENTING EQUIPMENT FROM ACINETOBACTER BAUMANNII CONTAMINATION**

(30) Priority: 29.10.2019 TW 108139082
(71) Applicant: Tzu Chi University, Hualien 97004 (TW)
(72) Inventor: Lin, Nien-Tsung, 97004 Hualien (TW)
(74) Representative: Lang, Christian

(57) **Abstract**

A use of a tail fiber protein for preventing equipment from Acinetobacter baumannii contamination is disclosed. The tail fiber protein obtained from bacteriophages is coated or sprayed on the equipment acting as carrier (such as pipelines and medical devices in hospitals) to inhibit biofilm formation of Acinetobacter baumannii and further prevent Acinetobacter baumannii infections associated with the use of the equipment.

<Sequence listing>
<110>   Tzu Chi University
<120>   USE OF A TAIL FIBER PROTEIN IN THE PREVENTION OF ACINETOBACTER
BAUMANNII CONTAMINATION
<130>   P14202EP00
<150>   TW108139082
<151>   2019-10-29
<160>   1
<210>   1
<211>   585
<212>   PRT
<213>   Bacteriophage
<400>   1
Met Gly Ser Ser His His His His His His Ser Ser Gly Leu Val Pro Arg Gly Ser
His Met Ser
1               5                   10                  15
20
Glu Ala Ala Gln Glu Ala Ala Asn Ala Ala Glu Val Ala Ala Ser Gln Thr Gln Tyr
Tyr Leu Lys
        25                  30                  35                  40

Tyr Phe Asn Pro Glu Ile Val Tyr Pro Lys Asn Ala Arg Ile Met Leu Asp Asn Gly
Asp Ile Val
45                  50                  55                  60
65
Arg Ser Thr Val Val Asn Asn Thr Ser Asn Pro Asn Val Asp Met Thr Gly Trp Val
Lys Val Ser
            70                  75                  80                  85
Ser Val Ser Gln Ile Phe Asp Glu Thr Tyr Asn Ile Thr Gln Ser Val Ile Asn Gly
Asn Leu Ile
    90                  95                  100                 105
110
Thr Val Asp Asn Phe Gly Ala Lys Gly Asp Gly Val Thr Asp Asp Ser Ala Ala Phe
Gln Ala
                115                 120                 125
130
Tyr Cys Asp Ser Ala Leu Thr Gly Gln Asn Leu Tyr Leu Gly Ala Lys Gly Arg Tyr
Ile Leu Lys
            135                 140                 145                 150
Asn Gln Val Asp Leu Lys Gly Lys Gly Leu Val Gly Asn Gly Cys Gly Lys Val Ser
Glu Phe Tyr
    155                 160                 165                 170
175
Tyr Asn Leu Gly Cys Ile Asp Val Asp Gly Ser Ser Pro Asp Leu Gln Gly Lys Thr
Ala Phe Ile
                180                 185                 190
195
Asn Cys Gly Pro Thr Ile Gln Asn Leu Thr Ala Arg Cys Ser Asn Gly Ala Gly Lys
Gln Val Ser
        200                 205                 210                 215

Phe Ile Glu Ile Asp Gly Tyr Leu Ala Asn Ile Asp His Ile Thr Leu Ile Asn Phe
Tyr Asn Gln
220                 225                 230                 235
240
Ile Val Val Lys Gln Ala Leu Val Gly Phe Asn Phe Thr Asn Ala Trp Leu Tyr Tyr
Ser Gln Asn
            245                 250                 255                 260

Ala Gly Ile Tyr Cys Glu Asp Pro Leu Asn Arg Val Ser Thr Thr Gly Thr Phe His
Asn Ile Tyr
    265                 270                 275                 280
285

Phe Gln Leu Gly Asp Gly His Ala Met Ile Phe Asp Arg Asp Val His Gly Cys Asp
Phe Asp
                290                 295                 300
305
Asn Ile Ile Phe Glu Ser Met Asn Gly Gly Ile Lys Ala Arg Thr Val Ala His Cys
Gly Phe Gly
            310                 315                 320                 325

Lys Phe Trp Cys Glu Asn Leu Lys Thr Ala Thr Ser Lys Asp Trp Leu Glu Val Thr
Gly Ala Asn
    330                 335                 340                 345
355
Ser Cys Tyr Gly Asn Ser Phe Thr Gly Tyr Val Lys Leu Leu Gly Gly Trp Thr Ser
Lys Thr Ser
                360                 365                 370
375
Pro Thr Leu Asp Ser Leu Pro Thr Asn Asn Tyr Gly Gly Val Ser Val Ser Ala Glu
Gly Ile Ser
        380                 385                 390                 395
Ile Val Asn Ala Gly Asn Lys Ala Lys Met Leu Met Leu Pro Ser Gly Phe Lys Thr
Gly Asn
400                 405                 410                 415
420
Ala Thr Ile Asp Glu Thr His Ile Ser Ser Ser Thr Val Thr Pro Leu Val Lys Arg
Arg Val Ile Gly
                425                 430                 435
440
Ala Asp Ser Ser Gly Ala Gln Tyr Leu Ala Ser Asp Thr Tyr Thr Lys Leu Ser Arg
Lys Trp Gly
    445                 450                 455                 460
465
Thr Tyr Asn His Gly Ser Asn Asn Ala Gly Ala Phe Tyr Ala Pro Met Met Leu Thr
Tyr Asp
                470                 475                 475
480
Gln Ser Phe Ser Thr Pro Gln Asn Asn Asn Gly Trp Lys Ile Val Lys Glu Ser Thr
Gly Val Tyr
                485                 490                 495                 500
Arg Val Glu Arg Val Ser Gly Asn Thr Ser Val Ile Thr Asn Gly His Ile Val Val
Gly Ser Pro
    505                 510                  515                 520
525
Leu Met Gly Ser Arg Leu Gly Thr Gly Thr Gly Ala Thr His Gly Ile Gln Met Ile
Glu Thr Tyr
                530                 535                 540
545
Ala Gly Ser Trp Thr Ser Tyr Thr Glu Ala Ala Gly Phe Lys Val Phe Trp Arg Asp
Ser Ser Asn
        550                 555                 560                 565
Ala Leu Val Asp Pro His Arg Phe Thr Val Ala Phe Thr Ala Thr Ser
570                 575                 580                 585

<Sequence listing>
<110>   Tzu Chi University
<120>   USE OF A TAIL FIBER PROTEIN IN THE PREVENTION OF ACINETOBACTER
BAUMANNII CONTAMINATION
<130>   P14202EP00
<150>   TW108139082
<151>   2019-10-29
<160>   3
<210>   1
<211>   585
<212>   PRT
<213>   Bacteriophage
<400>   1
Met Gly Ser Ser His His His His His His Ser Ser Gly Leu Val Pro
1               5                   10                  15
Arg Gly Ser His Met Ser Glu Ala Ala Gln Glu Ala Ala Asn Ala Ala
            20                  25                  30
Glu Val Ala Ala Ser Gln Thr Gln Tyr Tyr Leu Lys Tyr Phe Asn Pro
        35                  40                  45
Glu Ile Val Tyr Pro Lys Asn Ala Arg Ile Met Leu Asp Asn Gly Asp
    50                  55                  60
Ile Val Arg Ser Thr Val Val Asn Asn Thr Ser Asn Pro Asn Val Asp
65                  70                  75                  80
Met Thr Gly Trp Val Lys Val Ser Ser Val Ser Gln Ile Phe Asp Glu
                85                  90                  95
Thr Tyr Asn Ile Thr Gln Ser Val Ile Asn Gly Asn Leu Ile Thr Val
            100                 105                 110
Asp Asn Phe Gly Ala Lys Gly Asp Gly Val Thr Asp Asp Ser Ala Ala
        115                 120                 125
Phe Gln Ala Tyr Cys Asp Ser Ala Leu Thr Gly Gln Asn Leu Tyr Leu
    130                 135                 140
Gly Ala Lys Gly Arg Tyr Ile Leu Lys Asn Gln Val Asp Leu Lys Gly
145                 150                 155                 160
Lys Gly Leu Val Gly Asn Gly Cys Gly Lys Val Ser Glu Phe Tyr Tyr
                165                 170                 175
Asn Leu Gly Cys Ile Asp Val Asp Gly Ser Ser Pro Asp Leu Gln Gly
            180                 185                 190
Lys Thr Ala Phe Ile Asn Cys Gly Pro Thr Ile Gln Asn Leu Thr Ala
        195                 200                 205
Arg Cys Ser Asn Gly Ala Gly Lys Gln Val Ser Phe Ile Glu Ile Asp
    210                 215                 220
Gly Tyr Leu Ala Asn Ile Asp His Ile Thr Leu Ile Asn Phe Tyr Asn
225                 230                 235                 240
Gln Ile Val Val Lys Gln Ala Leu Val Gly Phe Asn Phe Thr Asn Ala
                245                 250                 255
Trp Leu Tyr Tyr Ser Gln Asn Ala Gly Ile Tyr Cys Glu Asp Pro Leu
            260                 265                 270
Asn Arg Val Ser Thr Thr Gly Thr Phe His Asn Ile Tyr Phe Gln Leu
        275                 280                 285
Gly Asp Gly His Ala Met Ile Phe Asp Arg Asp Val His Gly Cys Asp
    290                 295                 300
Phe Asp Asn Ile Ile Phe Glu Ser Met Asn Gly Gly Ile Lys Ala Arg
305                 310                 315                 320
Thr Val Ala His Cys Gly Phe Gly Lys Phe Trp Cys Glu Asn Leu Lys
                325                 330                 335
Thr Ala Thr Ser Lys Asp Trp Leu Glu Val Thr Gly Ala Asn Ser Cys
            340                 345                 350
Tyr Gly Asn Ser Phe Thr Gly Tyr Val Lys Leu Leu Gly Gly Trp Thr
        355                 360                 365
Ser Lys Thr Ser Pro Thr Leu Asp Ser Leu Pro Thr Asn Asn Tyr Gly
    370                 375                 380
Gly Val Ser Val Ser Ala Glu Gly Ile Ser Ile Val Asn Ala Gly Asn
385                 390                 395                 400

Lys Ala Lys Met Leu Met Leu Pro Ser Gly Phe Lys Thr Gly Asn Ala
                405                 410                 415
Thr Ile Asp Glu Thr His Ile Ser Ser Ser Thr Val Thr Pro Leu Val
            420                 425                 430
Lys Arg Arg Val Ile Gly Ala Asp Ser Ser Gly Ala Gln Tyr Leu Ala
        435                 440                 445
Ser Asp Thr Tyr Thr Lys Leu Ser Arg Lys Trp Gly Thr Tyr Asn His
    450                 455                 460
Gly Ser Asn Asn Ala Gly Ala Phe Tyr Ala Pro Met Met Leu Thr Tyr
465                 470                 475                 480
Asp Gln Ser Phe Ser Thr Pro Gln Asn Asn Asn Gly Trp Lys Ile Val
                485                 490                 495
Lys Glu Ser Thr Gly Val Tyr Arg Val Glu Arg Val Ser Gly Asn Thr
            500                 505                 510
Ser Val Ile Thr Asn Gly His Ile Val Val Gly Ser Pro Leu Met Gly
        515                 520                 525
Ser Arg Leu Gly Thr Gly Thr Gly Ala Thr His Gly Ile Gln Met Ile
    530                 535                 540
Glu Thr Tyr Ala Gly Ser Trp Thr Ser Tyr Thr Glu Ala Ala Gly Phe
545                 550                 555                 560
Lys Val Phe Trp Arg Asp Ser Ser Asn Ala Leu Val Asp Pro His Arg
                565                 570                 575
Phe Thr Val Ala Phe Thr Ala Thr Ser
            580                 585

<210>   2
<211>   27
<212>   DNA
<213>   Artificial Sequence
<220>
<223>   reverse primer
<400>   2
CTCGAGTTAA CTCGTTGCTG TAAATGC   27


<210>   3
<211>   32
<212>   DNA
<213>   Artificial Sequence
<220>
<223>   forward primer
<400>   3
CATATGAGTG AAGCTGCTGC TCAAGAGGCT GC      32

## Description

### BACKGROUND OF THE INVENTION

### Field of the Invention

The present invention relates to a use of a tail fiber protein, especially to a use of a tail fiber protein for preventing equipment from Acinetobacter baumannii contamination.

### Description of Related Art

Acinetobacter baumannii, abbreviated as AB or A. baumannii, is a rod-shaped Gram-negative bacterium with low motility due to lack of flagella. They are with strong vitality, naturally occurring in environmental matrices such as soil, water, food, etc. broadly and permanently, or even human skin or mucosa.

Acinetobacter baumannii can live in human bodies without causing infections or symptoms, and mainly colonize on skin, underarm, conjunctiva, perineum, oral cavity, upper respiratory tract and lower gastrointestinal tract, sometimes also in throat saliva and mucosa. These bacteria are present in the normal flora of about 10% health people.

Moreover, this highly-hydrophilic organism thrives in warm and wet environments. In addition to medical pipelines in the intensive care unit (ICU) including pipes of medical ventilators, Foley catheters, chest tubes, central venous catheters, surgical drainages, etc., Acinetobacter baumannii are commonly found on trolleys, sinks, beds and mattress, even air in the hospitals.

Most of scientists thaught that the organism is not pathogenic when Acinetobacter baumannii was isolated for the first time. Along with the fast development of the medical technology, various invasive catheters have been developed and used in critically ill patients. These catheters provide optimal environmental conditions to Acinetobacter baumannii and the critically ill patients have a weakened immune system. Thus, the frequency of Acinetobacter baumannii infections has been increased gradually.

Cultures of sputum, urine, blood and wound exudate are done for diagnosis of Acinetobacter baumannii in critically ill patients. Once the bacteria are detected in the patient's sputum, the positive result may result from a contaminated specimen and treatment is not necessary. Yet if Acinetobacter baumannii is observed in the urine, blood and wound exudate cultures, the result shows that the patients are infected. Especially a positive result in the blood culture represents that the bacteria already cause severe infections in patients, and even trigger lethal sepsis.

Risk factors for Acinetobacter baumannii infections include immune function of the patient, disease severity, etc. Thus, the more serious the illness, the longer hospital stays, with medical ventilators, and the more catheters used, the greater frequency of infections in patients. Thereby the critically ill patients in the intensive care unit (ICU) are at high risk for nosocomial infection.

In healthy people, Acinetobacter baumannii is a common flora. Acinetobacter baumannii can be spread by person-to-person contact or contact with contaminated surfaces of general medical devices. Although not as horrifying as droplet transmission, Acinetobacter baumannii is still easily transmitted once medical professionals forget to clean their hands before in contact with the next patient.

Acinetobacter baumannii, infection may cause bacteremia, sepsis, pneumonia, meningitis, peritonitis, endocarditis, urinary tract infection, skin infection, etc., sometimes even cause death of patients. The mortality rate of patients with Acinetobacter baumannii, have been reported to be as highly as 20∼50%.

Furthermore, higher antibiotic resistance rates for Acinetobacter baumannii are resulted from the abuse of antibiotics. The use of antibiotics should be reframed. In recent years, more and more antibiotic resistant strains of antibiotic resistant strains are appearing and this poses a greater challenge in treatment of patients with these antibiotic resistant strains.

For Acinetobacter baumannii nosocomial infection, complete infection control and prevention are far more important than the treatment. Thus, there is a room for improvement and there is a need to provide a novel way for prevention of Acinetobacter baumannii infections caused by Acinetobacter baumannii in general medical devices.

### SUMMARY OF THE INVENTION

Therefore, it is a primary object of the present invention to provide a use of a tail fiber protein for preventing equipment from Acinetobacter baumannii contamination. The tail fiber protein derived from bacteriophage φAB6 is disposed on carriers such as pipelines and medical devices in hospitals, to degrade, inhibit and prevent biofilm formation of Acinetobacter baumannii on the carrier and thereby further prevent Acinetobacter baumannii infections of patients.

In order to achieve the above object, a use of a tail fiber protein for preventing equipment from Acinetobacter baumannii contamination according to claim 1 of the present invention is provided. The amino acid sequence of the tail fiber protein is set forth in SED ID NO : 1 and the tail fiber protein is disposed on carriers defined by the equipment to be treated to prevent Acinetobacter baumannii contamination thereof and thereby further preventing Acinetobacter baumannii infections associated with the carriers. Preferred embodiments are defined in the dependent claims.

Preferably, regarding the use of the tail fiber protein for preventing equipment from Acinetobacter baumannii contamination, the tail fiber protein is obtained from a bacteriophage.

Preferably, regarding the use of the tail fiber protein for preventing equipment from Acinetobacter baumannii contamination, the Acinetobacter baumannii is Acinetobacter baumannii strain 54149.

Preferably, regarding the use of the tail fiber protein for preventing equipment from Acinetobacter baumannii contamination, the tail fiber protein is able to degrade a biofilm of Acinetobacter baumannii.

Preferably, regarding the use of the tail fiber protein for preventing equipment from Acinetobacter baumannii contamination, the tail fiber protein is able to inhibit biofilm formation of Acinetobacter baumannii.

Preferably, regarding the use of the tail fiber protein for preventing equipment from Acinetobacter baumannii contamination, the tail fiber protein is able to reduce thickness of a biofilm of Acinetobacter baumannii.

Preferably, regarding the use of the tail fiber protein for preventing equipment from Acinetobacter baumannii contamination, the tail fiber protein is disposed on the carrier by coating.

Preferably, regarding the use of the tail fiber protein for preventing equipment from Acinetobacter baumannii contamination, the tail fiber protein is arranged on the carrier by spraying.

Preferably, regarding the use of the tail fiber protein for preventing equipment, such as a carrier, from Acinetobacter baumannii contamination, , the carrier is selected from one of the followings: a pipe of the ventilator, a Foley catheter, a chest tube, a central venous catheter, and a surgical drainage.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein:
Fig. 1 is a bar chart showing biofilm degradation results of an embodiment according to the present invention;
Fig. 2 is a bar chart showing biofilm inhibition effect of an embodiment according to the present invention;
Fig. 3 shows images showing biofilm thickness of an embodiment according to the present invention;
Fig. 4A-4B show experiment results of an embodiment according to the present invention.

### DETAILED DESCRIPTION OF THE PREFERRED EMBODIMENT

In order to better understand the features and functions of the present invention, please refer to the following embodiments and related figures.

Acinetobacter baumannii infections typically occur in people in hospitals and even threaten patient's lives. In view of this, the present invention provides a use of a tail fiber protein for preventing equipment from Acinetobacter baumannii contamination to solve the problems caused by the infections associated with contaminated equipment.

The features, related structure and methods used are exemplarily described by means of the following embodiments.

The tail fiber protein (abbreviated as TFP) for use according to the present invention is derived from bacteriophage φAB6 and has an amino acid sequence as set forth in SED ID NO : 1 as below:

MGSSHHHHHHSSGLVPRGSHMSEAAQEAANAAEVAASQTQYYL
KYFNPEIVYPKNARIMLDNGDIVRSTVVNNTSNPNVDMTGWVKV
SSVSQIFDETYNITQSVINGNLITVDNFGAKGDGVTDDSAAFQAYC
DSALTGQNLYLGAKGRYILKNQVDLKGKGLVGNGCGKVSEFYY
NLGCIDVDGSSPDLQGKTAFINCGPTIQNLTARCSNGAGKQVSFIEI
DGYLANIDHITLINFYNQIVVKQALVGFNFTNAWLYYSQNAGIYC
EDPLNRVSTTGTFHNIYFQLGDGHAMIFDRDVHGCDFDNIIFESMN
GGIKARTVAHCGFGKFWCENLKTATSKDWLEVTGANSCYGNSFT
GYVKLLGGWTSKTSPTLDSLPTNNYGGVSVSAEGISIVNAGNKAK
MLMLPSGFKTGNATIDETHISSSTVTPLVKRRVIGADSSGAQYLAS
DTYTKLSRKWGTYNHGSNNAGAFYAPMMLTYDQSFSTPQNNNG

WKIVKESTGVYRVERVSGNTSVITNGHIVVGSPLMGSRLGTGTGA
THGIQMIETYAGSWTSYTEAAGFKVFWRDSSNALVDPHRFTVAFT
ATS.

The tail fiber protein is further disposed on a surface of a carrier, which is at least part of a medical equipment, to prevent the carrier from being contaminated with Acinetobacter baumannii. In this embodiment, the Acinetobacter baumannii is mild antibiotic -resistant Acinetobacter baumannii strain 54149. The tail fiber protein is obtained by using Polymerase chain reaction (PCR). The purified ORF40φAB6 segment in bacteriophage (φAB6) with polysaccharide depolymerase activity is amplified and extracted while the primers used include XhoI_ABTF6_R (5'-CTCGAGTTAACTCGTTGCTGTAAATGC-3') and NdeI_ABTF6_F (5'-CATATGAGTGAAGCTGCTGCTCAAGAGGCTGC-3'). Next the segment cut by restriction enzymes XhoI and NdeI is inserted into the pET-28a vector and the pET-28a vector is delivered into Escherichia coli BL21 (DE3). Lastly the protein obtained is analyzed and purified.

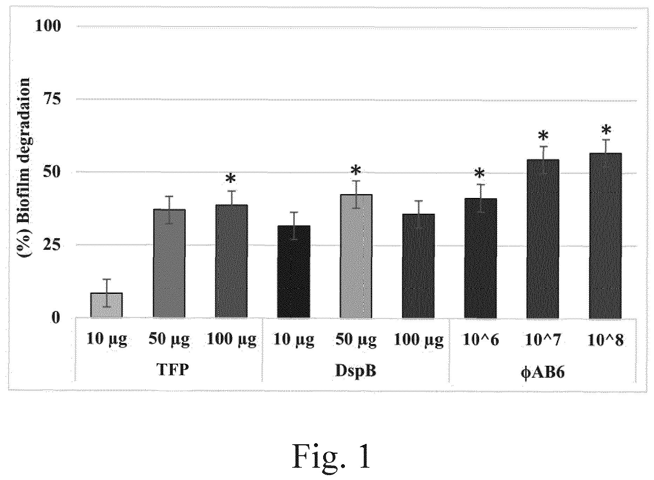

Refer to Fig. 1, which reveals a bar chart showing biofilm degradation results of an embodiment. A sterile polystyrene microtiter plate (microplate) with 96 wells is used to perform biofilm degradation tests. Acinetobacter baumannii strain 54149 is cultured in the microplate with culture medium for 48 hours. After bacterial suspension (Acinetobacter baumannii strain 54149 and culture medium) being removed, bacteriophage (φAB6) at different concentrations (10⁶, 10⁷, and 10⁸ PFU), the tail fiber protein (TFP) at different concentrations (10, 50, and 100 µg) and DspB at different concentrations (10, 50, and 100 µg) are respectively added into the microplate and incubated at 37°C, 150rpm for 4 hours. DspB, also known as Dispersin B, is an anti-biofilm agent that could be used in combination with antibiotics for the treatment of bacterial infections. Next step is washing gently with IX phosphate buffered saline (PBS) and stain with 200µl, 0.1% crystal violet solution for 30 minutes. Then remove the crystal violet solution with 1X PBS and let the microplate dry at 55°C for 30 minutes. Lastly 95% alcohol are added for dissolution, the microplate is shaken for 10 minutes and biofilm degradation is determined by measuring absorbance of both attached cells and unattached cells (using a ELISA reader at OD 570nm). The average absorbance of the respective groups (the bacteriophage, the tail fiber protein, and DspB) being processed is divided by the average absorbance of the control wells and then is multiplied by 100 to learn the biofilm degradation effect (biofilm degradation rate).

As shown in Fig. 1, the biofilm degradation rate of the tail fiber protein (TFP) at the concentration of 100µg is up to 36% (p <0.05).

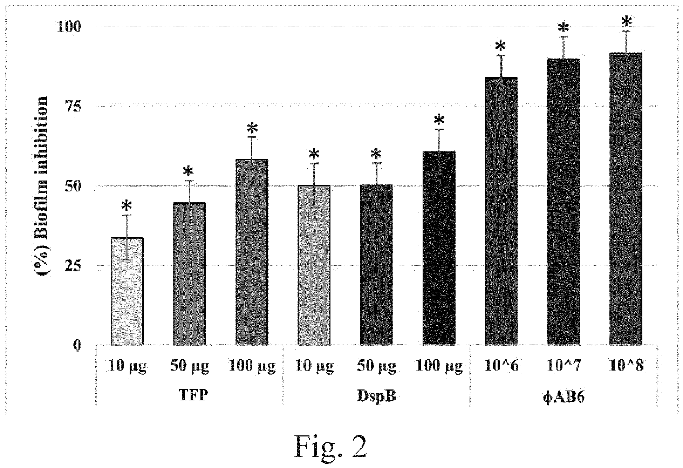

Refer to Fig. 2, a bar chart showing biofilm inhibition effect of an embodiment is disclosed. Biofilm inhibition tests are carried out by using the same sterile polystyrene microtiter plate (microplate) with 96 wells mentioned above. Bacteriophage (φAB6) at different concentrations (10⁶, 10⁷, and 10⁸ PFU), the tail fiber protein (TFP) at different concentrations (10, 50, and 100 µg), DspB at different concentrations (10, 50, and 100 µg) and Acinetobacter baumannii strain 54149 (abbreviated as Ab-54149) are respectively added into and incubated in the microplate and incubated for 24 hours. Then the biofilm inhibition effect is evaluated by measuring absorbance of unattached cells (at OD 570nm in an ELISA reader). The biofilm inhibition rate is calculated by the average absorbance of respective groups (the bacteriophage, the tail fiber protein, and DspB) processed being divided by the average absorbance of the control wells and then being multiplied by 100.

As shown in Fig. 2, the tail fiber protein (TFP) significantly inhibits the biofilm formation of the Acinetobacter baumannii strain 54149 (p <0.01) and the biofilm inhibition rate at the concentration of 100µg is up to 60% (p <0.05). Moreover, the biofilm inhibition effect is proportional to the concentration of the tail fiber protein (TFP). The results show that the tail fiber protein (TFP) has a great ability in inhibition of biofilm formation.

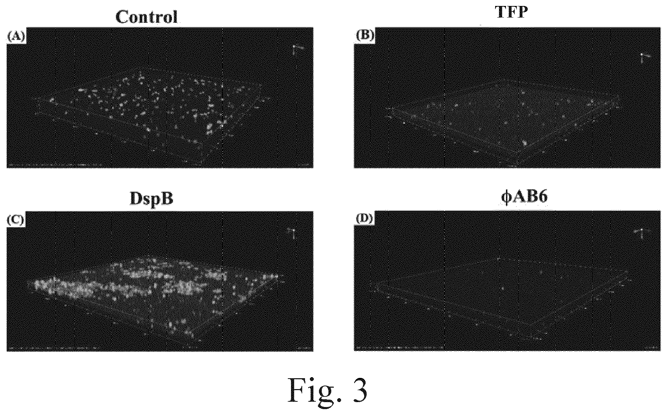

Refer to Fig. 3, images showing biofilm thickness are disclosed. A confocal Laser Scanning Microscope (CLSM) is used to observe the 3-dimensional structure of the biofilm for testing a reduction of biofilm thickness. The biofilm of the Acinetobacter baumannii strain 54149 is cultured on round plastic coverslips in a 24-well microplate. Then washing the round plastic coverslips twice with IX PBS and add the bacteriophage (φAB6, 10⁸ PFU in PBS), the tail fiber protein (TFP, 100 µg in PBS) and DspB (100 µg in PBS) into the plastic coverslips to be incubated at 37°C, 150rpm for 4 hours. After being washed with IX PBS, stain in a dark room with wheat germ agglutinin-Alexa Fluor (WGA-AF) for 2 hours. The Alexa Fluor can bind to extracellular polymeric substances (EPS) to emit green fluorescence. At last, CLSM is used to observe the biofilm after washing with PBS.

Referring to Fig. 3 biofilm thickness reduction is shown. Compared to the control group (Group A, the tail fiber protein is inactivated), the thickness of the biofilm in the group B being processed by the tail fiber protein is significantly reduced. Moreover, compared to the present invention (group B), the thickness of the biofilm of Acinetobacter baumannii strain 54149 on the cover slip of the group treated by DspB (Group C) is not reduced effectively.

Furthermore, the present tail fiber protein is disposed on a carrier by coating and the carrier is equipment selected from, but not limited to, one of the followings: a pipe of the ventilator, a Foley catheter, a chest tube, a central venous catheter, and a surgical drainage. The present tail fiber protein can also be arranged at the carrier by spraying. Besides the pipelines mentioned above, the carrier may also include other medical devices used in hospitals, such as wheelchairs, beds, trolleys, door handles, etc. The tail fiber protein is disposed over the devices by spraying to prevent biofilm formation of Acinetobacter baumannii strain 54149 and related infections.

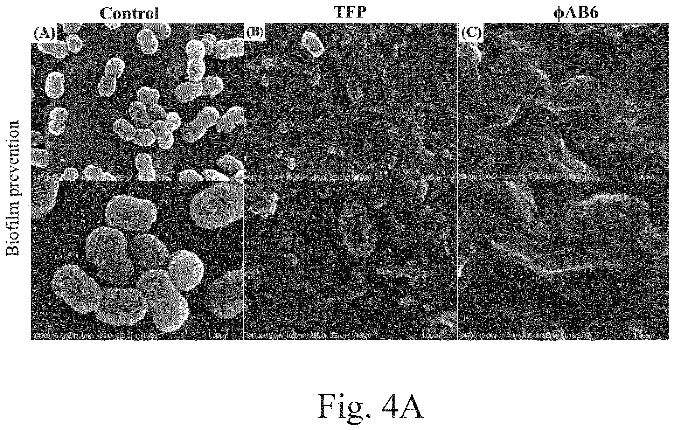

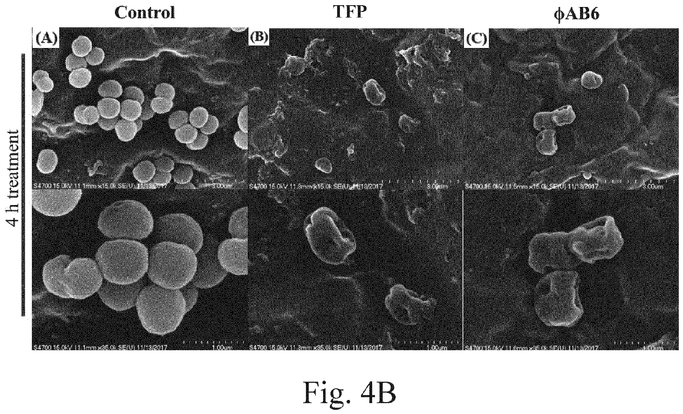

Refer to Fig. 4A and Fig. 4B, experiment results of an embodiment of the present invention are revealed. A scanning electron microscope (SEM) is used to observe the biofilm on a catheter (Foley catheter with the silicon coating). Three Foley catheters with the silicon coating are respectively immersed in 1ml IX PBS (Group A as the control group), 1mg tail fiber protein (Group B, TFP) and 10⁸ PFU bacteriophage (Group C, φAB6) for 16 hours. Then the Foley catheters with the silicon coating are immersed in bacteria suspension (Acinetobacter baumannii strain 54149+culture medium). Next wash twice with IX PBS and use 2.5% (w/v) Glutaraldehyde in 01.M cacodylate buffer and 1% aqueous osmium tetroxide for fixation. Then wash twice with IX PBS again and dehydrate in a graded ethanol series (50%, 70% and 95%). Lastly perform chemical drying by using 100% Hexamethyldisilazane (HMDS). Then the scanning electron microscope (SEM) is used to observe the results of the experiment designed to test the prevention effect of the tail fiber protein on biofilm formation of Acinetobacter baumannii strain 54149 on the Foley catheter with silicon coating.

As shown in Fig. 4A, Acinetobacter baumannii strain 54149 is unable to grow on the catheters already being immersed in the tail fiber protein (TFP) and the bacteriophage (φAB6). Thereby the results indicate that the tail fiber protein (TFP) can prevent both the growth of Acinetobacter baumannii strain 54149 and its biofilm formation.

Refer to Fig. 4B, the experiment results that show biofilm degradation effect of the tail fiber protein (TFP) on Acinetobacter baumannii strain 54149 on the catheter are disclosed. After being immersed in bacteria suspension (Acinetobacter baumannii strain 54149+culture medium) at 37 °C for 48 hours, the Foley catheters with the silicon coating are respectively further treated by IX PBS(Group A as the control group), 1mg tail fiber protein (Group B, TFP) and 10⁸ PFU bacteriophage (Group C, φAB6) for 4 hours. The same as the experiment in Fig. 4A, the scanning electron microscope (SEM) is used to observe the biofilm. As shown in Fig. 4B, the tail fiber protein (TFP) degrades the biofilm of Acinetobacter baumannii strain 54149 to prevent Acinetobacter baumannii strain 54149 from growing.

According to the above experiments, it is proved that the tail fiber protein (TFP) can not only prevent biofilm formation of Acinetobacter baumannii strain 54149 on catheters or other medical devices and related infections, the tail fiber protein (TFP) can also degrade the biofilm already formed (by Acinetobacter baumannii strain 54149) and stop the growth of Acinetobacter baumannii strain 54149.

The tail fiber protein of the present invention prevents contamination with the Acinetobacter baumannii strain 54149 by inhibiting biofilm formation of Acinetobacter baumannii strain 54149 on the carrier. In the conventional way, bacteriophages which directly lyse and burst Acinetobacter baumannii strain 54149 are used so that bacterial endotoxin is released into human bodies through the carrier and causing a wide range of adverse effects on the bodies. The way the present invention uses is quite different from the conventional way.

In summary, besides reducing attachment and colonization of Acinetobacter baumannii strain 54149 on the carrier, the present tail fiber protein also reduces antibiotic tolerance of the bacteria strain and solves the problem of endotoxin release while Acinetobacter baumannii strain 54149 being killed.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalent.

## Claims

1. A use of a tail fiber protein for preventing equipment from Acinetobacter baumannii contamination; wherein the tail fiber protein has an amino acid sequence as set forth in SED ID NO: 1 and the tail fiber protein is disposed on a carrier to prevent Acinetobacter baumannii infections associated with the carrier.

2. The use as claimed in claim 1, wherein the tail fiber protein is obtained from a bacteriophage.

3. The use as claimed in claim 1, wherein the Acinetobacter baumannii is Acinetobacter baumannii strain 54149.

4. The use as claimed in claim 1, wherein the tail fiber protein degrades a biofilm of Acinetobacter baumannii.

5. The use as claimed in claim 1, wherein the tail fiber protein inhibits biofilm formation of Acinetobacter baumannii.

6. The use as claimed in claim 1, wherein the tail fiber protein reduces thickness of a biofilm of Acinetobacter baumannii.

7. The use as claimed in claim 1, wherein the tail fiber protein is disposed on the carrier by coating.

8. The use as claimed in claim 1, wherein the tail fiber protein is arranged at the carrier by spraying.

9. The use as claimed in claim 1, wherein the carrier is selected from the group consisting of a pipe of the ventilator, a Foley catheter, a chest tube, a central venous catheter, and a surgical drainage.
